# EUROPEAN PATENT SPECIFICATION

(11) **EP 0 980 526 B1**
(45) Date of publication and mention of the grant of the patent: **30.08.2006**
(21) Application number: 98919329.7
(22) Date of filing: 28.04.1998
(51) Int. Cl.: G01N 33/68, C07K 7/06, C07K 7/08

(54) **INHIBITORS OF NUCLEAR PROTEIN/NUCLEAR RECEPTOR INTERACTION**
INHIBITOREN DER WECHSELWIRKUNG ZWISCHEN NUKLEÄRE PROTEIN UND NUKLEÄREN REZEPTOREN
INHIBITEURS DE L'INTERACTION RECEPTEUR NUCLEAIRE/PROTEINE NUCLEAIRE

(30) Priority: 30.04.1997 GB 9708676
(43) Date of publication of application: 23.02.2000
(73) Proprietor: IMPERIAL CANCER RESEARCH TECHNOLOGY LIMITED, London WC2A 3NL (GB)
(72) Inventor: HEERY, David, Michael, Imperial Cancer Res. Fund, London WC2A 3PX (GB); PARKER, Malcolm, George, Imperial Cancer Res. Fund, London WC2A 3PX (GB)
(74) Representative: Giles, Allen Frank
(86) International application number: PCT/GB1998/001238
(87) International publication number: WO 1998/049561

(56) References cited:
- WO-A-97/45737
- WO-A-98/02455
- WO-A-98/11907

### SEQUENCE LISTING

(1) GENERAL INFORMATION:
   (i) APPLICANT:
      (A) NAME: Imperial Cancer Research Technology Limited
      (B) STREET: Sardinia House, Sardinia Street,
      (C) CITY: London
      (D) STATE: England
      (E) COUNTRY: Great Britain
      (F) POSTAL CODE (ZIP): WC2A 3NL
      (G) TELEPHONE: 0171 242 1136
      (H) TELEFAX: 0171 831 4991
   (ii) TITLE OF INVENTION: Chemical Compounds
   (iii) NUMBER OF SEQUENCES: 79
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: GB 9708676.3
      (B) FILING DATE: 30-APR-1997
(2) INFORMATION FOR SEQ ID NO: 1:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 1:
   Leu Xaa Xaa Leu Leu
   1               5
(2) INFORMATION FOR SEQ ID NO: 2:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 2:
   Pro Gln Ala Gln Gln Lys Ser Leu Leu Gln Gln Leu Leu Thr
   1               5                   10
(2) INFORMATION FOR SEQ ID NO: 3:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 3:
   Lys Leu Val Gln Leu Leu Thr Thr Thr
   1               5
(2) INFORMATION FOR SEQ ID NO: 4:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 4:
   Ile Leu His Arg Leu Leu Gln Glu
   1               5
(2) INFORMATION FOR SEQ ID NO: 5:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 5:
   Leu Leu Gln Gln Leu Leu Thr Glu
   1               5
(2) INFORMATION FOR SEQ ID NO: 6:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 6:
   Tyr Leu Glu Gly Leu Leu Met His Gln Ala
   1               5                   10
(2) INFORMATION FOR SEQ ID NO: 7:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 7:
   Leu Leu Ala Ser Leu Leu Gln Ser
   1               5
(2) INFORMATION FOR SEQ ID NO: 8:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 8:
   His Leu Lys Thr Leu Leu Lys Lys Ser
   1               5
(2) INFORMATION FOR SEQ ID NO: 9:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 9:
   Gln Leu Ala Leu Leu Leu Ser Ser
   1               5
(2) INFORMATION FOR SEQ ID NO: 10:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 10:
   Leu Leu Leu His Leu Leu Lys Ser Gln
   1               5
(2) INFORMATION FOR SEQ ID NO: 11:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 11:
   Val Thr Leu Leu Gln Leu Leu Leu Gly
   1               5
(2) INFORMATION FOR SEQ ID NO: 12:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 12:
   Val Leu Gln Leu Leu Leu Gly Asn
   1               5
(2) INFORMATION FOR SEQ ID NO: 13:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 13:
   Leu Leu Ser Arg Leu Leu Arg Gln
   1               5
(2) INFORMATION FOR SEQ ID NO: 14:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 14:
   Val Leu Lys Gln Leu Leu Leu Ser Glu Asn
   1               5                   10
(2) INFORMATION FOR SEQ ID NO: 15:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 15:
   Val Leu Lys Gln Leu Leu Leu Ser
   1               5
(2) INFORMATION FOR SEQ ID NO: 16:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 16:
   Glu Leu Ala Glu Leu Leu Ser Ala Asn
   1               5
(2) INFORMATION FOR SEQ ID NO: 17:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 17:
   Ser Leu Gly Pro Leu Leu Leu Glu
   1               5
(2) INFORMATION FOR SEQ ID NO: 18:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 18:
   Lys Leu Val Gln Leu Leu Thr Thr Thr
   1               5
(2) INFORMATION FOR SEQ ID NO: 19:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 19:
   Ile Leu His Arg Leu Leu Gln Glu
   1               5
(2) INFORMATION FOR SEQ ID NO: 20:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 20:
   Leu Leu Arg Tyr Leu Leu Asp Lys
   1               5
(2) INFORMATION FOR SEQ ID NO: 21:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 21:
   Gln Leu Asp Glu Leu Leu Cys Pro Pro
   1               5
(2) INFORMATION FOR SEQ ID NO: 22:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 22:
   Leu Leu Gln Gln Leu Leu Thr Glu
   1               5
(2) INFORMATION FOR SEQ ID NO: 23:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 23:
   Gln Leu Ser Glu Leu Leu Arg Gly
   1               5
(2) INFORMATION FOR SEQ ID NO: 24:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 24:
   Val Leu Lys Gln Leu Leu Leu Ser Glu Asn
   1               5                   10
(2) INFORMATION FOR SEQ ID NO: 25:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 25:
   Ala Leu Lys Gln Leu Leu Leu Ser Glu
   1               5
(2) INFORMATION FOR SEQ ID NO: 26:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 26:
   Val Ala Lys Gln Leu Leu Leu Ser Glu
   1               5
(2) INFORMATION FOR SEQ ID NO: 27:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 27:
   Val Val Lys Gln Leu Leu Leu Ser Glu
   1               5
(2) INFORMATION FOR SEQ ID NO: 28:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 28:
   Val Leu Lys Gln Ala Leu Leu Ser Glu
   1               5
(2) INFORMATION FOR SEQ ID NO: 29:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 29:
   Val Leu Lys Gln Val Leu Leu Ser Glu
   1               5
(2) INFORMATION FOR SEQ ID NO: 30:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 30:
   Val Leu Lys Gln Leu Ala Leu Ser Glu
   1               5
(2) INFORMATION FOR SEQ ID NO: 31:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 31:
   Val Leu Lys Gln Leu Val Leu Ser Glu
   1               5
(2) INFORMATION FOR SEQ ID NO: 32:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 32:
   Val Leu Lys Gln Leu Leu Ala Ser Glu
   1               5
(2) INFORMATION FOR SEQ ID NO: 33:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 33:
   Tyr Leu Glu Gly Leu Leu Met His Gln Ala Ala
   1               5                   10
(2) INFORMATION FOR SEQ ID NO: 34:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 34:
   Leu Leu Ala Ser Leu Leu Gln Ser Glu Ser Ser
   1               5                   10
(2) INFORMATION FOR SEQ ID NO: 35:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 35:
   His Leu Lys Thr Leu Leu Lys Lys Ser Lys Val
   1               5                   10
(2) INFORMATION FOR SEQ ID NO: 36:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 36:
   Gln Leu Ala Leu Leu Leu Ser Ser Glu Ala His
   1               5                   10
(2) INFORMATION FOR SEQ ID NO: 37:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 37:
   Leu Leu Leu His Leu Leu Lys Ser Gln Thr Ile
   1 5 10
(2) INFORMATION FOR SEQ ID NO: 38:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 38:
   Leu Leu Gln Leu Leu Leu Gly His Lys Asn Glu
   1 5 10
(2) INFORMATION FOR SEQ ID NO: 39:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 39:
   Val Leu Gln Leu Leu Leu Gly Asn Pro Lys Gly
   1 5 10
(2) INFORMATION FOR SEQ ID NO: 40:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 40:
   Leu Leu Ser Arg Leu Leu Arg Gln Asn Gln Asp
   1 5 10
(2) INFORMATION FOR SEQ ID NO: 41:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 41:
   Val Leu Lys Gln Leu Leu Leu Ser Glu Asn Cys
   1               5                   10
(2) INFORMATION FOR SEQ ID NO: 42:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 42:
   Lys Leu Val Gln Leu Leu Thr Thr Thr Ala Glu
   1               5                   10
(2) INFORMATION FOR SEQ ID NO: 43:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 43:
   Ile Leu His Arg Leu Leu Gln Glu Gly Ser Pro
   1               5                   10
(2) INFORMATION FOR SEQ ID NO: 44:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 44:
   Leu Leu Arg Tyr Leu Leu Asp Lys Asp Glu Lys
   1               5                   10
(2) INFORMATION FOR SEQ ID NO: 45:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 45:
   Leu Leu Gln Gln Leu Leu Thr Glu
   1               5
(2) INFORMATION FOR SEQ ID NO: 46:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 46:
   Lys Leu Leu Gln Leu Leu Thr Thr Lys Ser Asp
   1               5                   10
(2) INFORMATION FOR SEQ ID NO: 47:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 47:
   Ile Leu His Arg Leu Leu Gln Asp Ser Ser Ser
   1               5                   10
(2) INFORMATION FOR SEQ ID NO: 48:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 48:
   Leu Leu Arg Tyr Leu Leu Asp Lys Asp Asp Thr
   1               5                   10
(2) INFORMATION FOR SEQ ID NO: 49:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 49:
   Gln Leu Ser Glu Leu Leu Arg Gly Gly Ser Gly
   1               5                   10
(2) INFORMATION FOR SEQ ID NO: 50:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 50:
   Gln Leu Val Leu Leu Leu His Ala His Lys Cys
   1               5                   10
(2) INFORMATION FOR SEQ ID NO: 51:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 51:
   Gln Leu Ser Glu Leu Leu Arg Gly Ser Ser Pro
   1 5 10
(2) INFORMATION FOR SEQ ID NO: 52:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 52:
   Gln Leu Val Leu Leu Leu His Ala His Lys Cys
   1 5 10
(2) INFORMATION FOR SEQ ID NO: 53:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 53:
   Ile Leu Thr Ser Leu Leu Leu Asn Ser Ser Gln
   1 5 10
(2) INFORMATION FOR SEQ ID NO: 54:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 54:
   Met Leu Met Asn Leu Leu Lys Asp Asn Pro Ala
   1 5 10
(2) INFORMATION FOR SEQ ID NO: 55:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 55:
   Thr Leu Arg Ser Leu Leu Leu Asn Pro His Leu
   1               5                   10
(2) INFORMATION FOR SEQ ID NO: 56:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 56:
   Arg Leu Ala Val Leu Leu Pro Gly Arg His Pro
   1               5                   10
(2) INFORMATION FOR SEQ ID NO: 57:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 57:
   Glu Leu His Asn Leu Leu Glu Val Val Ser Gln
   1               5                   10
(2) INFORMATION FOR SEQ ID NO: 58:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 58:
   Thr Leu Arg Asp Leu Leu Thr Thr Thr Ala Gly
   1               5                   10
(2) INFORMATION FOR SEQ ID NO: 59:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 59:
   Phe Leu Asp Phe Leu Leu Gly Phe Ser Ala Gly
   1               5                   10
(2) INFORMATION FOR SEQ ID NO: 60:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 60:
   Val Leu Glu Leu Leu Leu Arg Ala Gly Ala Asn
   1               5                   10
(2) INFORMATION FOR SEQ ID NO: 61:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 61:
   Ile Leu Ala Arg Leu Leu Arg Ala His Gly Ala
   1               5                   10
(2) INFORMATION FOR SEQ ID NO: 62:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 62:
   Ala Leu Gln Asp Leu Leu Arg Thr Leu Lys Ser
   1               5                   10
(2) INFORMATION FOR SEQ ID NO: 63:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 63:
   Ala Leu Gln Asn Leu Leu Arg Thr Leu Arg Ser
   1               5                   10
(2) INFORMATION FOR SEQ ID NO: 64:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 64:
   Lys Leu Leu Gln Leu Leu Thr Cys Ser Ser Asp
   1               5                   10
(2) INFORMATION FOR SEQ ID NO: 65:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 65:
   Ile Leu His Lys Leu Leu Gln Asn Gly Asn Ser
   1               5                   10
(2) INFORMATION FOR SEQ ID NO: 66:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 66:
   Leu Leu Arg Tyr Leu Leu Asp Arg Asp Asp Pro
   1               5                   10
(2) INFORMATION FOR SEQ ID NO: 67:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 67:
   Gln Leu Tyr Ser Leu Leu Gly Gln Phe Asn Cys
   1               5                   10
(2) INFORMATION FOR SEQ ID NO: 68:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 68:
   Glu Leu Glu Asn Leu Leu Gln Gln Gly Gly
   1               5                   10
(2) INFORMATION FOR SEQ ID NO: 69:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 69:
   Val Leu Gln Lys Leu Leu Lys Glu Lys Asp
   1 5 10
(2) INFORMATION FOR SEQ ID NO: 70:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 70:
   Glu Leu Asn Gln Leu Leu Asn Ala Val Lys
   1 5 10
(2) INFORMATION FOR SEQ ID NO: 71:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 71:
   Val Leu Lys Asp Leu Leu Lys Gln
   1 5
(2) INFORMATION FOR SEQ ID NO: 72:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 72:
   Pro Gln Ala Gln Gln Lys Ser Leu Leu Gln Gln Ala Ala Thr
   1 5 10
(2) INFORMATION FOR SEQ ID NO: 73:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 73:
   Ser Gln Thr Ser His Lys Leu Val Gln Leu Leu Thr Thr Thr
   1               5                   10
(2) INFORMATION FOR SEQ ID NO: 74:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 74:
   Thr Ala Arg His Lys Ile Leu His Arg Leu Leu Gln Glu
   1               5                   10
(2) INFORMATION FOR SEQ ID NO: 75:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 75:
   Ser Lys Asp His Gln Leu Leu Arg Tyr Leu Leu Asp Lys
   1               5                   10
(2) INFORMATION FOR SEQ ID NO: 76:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 76:
   Gln Ala Gln Gln Lys Ser Leu Leu Gln Gln Leu Leu Thr Glu
   1               5                   10
(2) INFORMATION FOR SEQ ID NO: 77:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 77:
      GAATTCCTGC AGCCCGGGGT CGACACTAGT TAACTAGCGG CCGC 44
(2) INFORMATION FOR SEQ ID NO: 78:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 78:
      AAGCTTCCAC CATGGTGCCA AAGAAGAAAC GTAAAGTT 38
(2) INFORMATION FOR SEQ ID NO: 79:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 79:
      TCTAGACTGC AGACTAGTAG ATCTCCCGGG GCGGCCGC 38
(2) INFORMATION FOR SEQ ID NO: 80:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 80:
   Glu Phe Leu Gln Pro Gly Val Asp Thr Ser
   1               5                   10
(2) INFORMATION FOR SEQ ID NO: 81:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 81:
   Met Val Pro Lys Lys Lys Arg Lys Val
   1               5

## Description

The present invention relates to inhibition of the interaction between nuclear proteins and nuclear receptors through identification of the key structural element responsible for the interaction.

The binding of lipophilic hormones, retinoids and vitamins to members of the nuclear receptor (NR) superfamily (to form "liganded'' receptors) modifies their DNA binding and transcriptional properties, resulting in the activation or repression of target genes ^{1.2}. Ligand binding induces conformational changes in NRs and promotes their association with a diverse group of nuclear proteins, including SRC-1/p160 3.4.5. TIF2 ^{6.7} and CBP/p300 ^{4.5.8.9} which function as coactivators, and RIP-140 ¹⁰, TIFI¹¹ and TRIP1/SUG1 ^{12. 13} whose functions are unclear.

The recruitment of nuclear proteins (coactivators and/or other so-called bridging proteins) by NRs is thought to be essential to their function as ligand-induced transcription factors. Structural studies of the ligand binding domains (LBDs) of three different nuclear hormone receptors, the retinoid X receptor α (RXRα)¹⁵. the retinoic acid receptor γ (RARγ) 16 and the thyroid hormone receptor β (TRβ) ¹⁷, have led to the proposal that binding of ligand results in a realignment of a conserved amphipathic α-helix. Helix 12 (H12), generating a novel surface required for coactivator binding and consequently activator function 2 (AF2)-dependent transactivation. Consistent with this. mutations of conserved hydrophobic residues in H12 which impair AF2 ^{14. 18-20}. also interfere with the ability of NRs to bind coactivators ^{4.6.10.11.13}. Less is known about the coactivator sequences which mediate interaction with NRs although several proteins appear to contain multiple NR binding sites ^{5.8.21.} Le Douarin *et al* (1996) in EMBO Journal, 15, 6701-6715, identified a leucine rich region in three coactivators (TIF1, RIP140 & TRIP3) which they called the "NR box"; see Figure 3D therein. However the present state of knowledge is completely silent about precisely how liganded nuclear receptors interact with nuclear proteins as a class to modify their DNA binding and transcriptional properties, resulting in the activation or repression of target genes. Indeed a commentator on the field stated, after the first filing date of the present invention, that *"characterizing the mechanisms by which nuclear factors engage the transcriptional apparatus in response to hormonal stimulation has seemed. at times, to be an insurmountable task"* (Marc Montminy in Nature, 12^{th} June, 1997, 387, 654-655, see 1^{st} paragraph thereof). WO 98/11907 relates to a localisation sequence found on a nuclear receptor. WO 97/45737 relates to a nuclear hormone receptor having an unknown ligand. WO 98/02455, which falls under Art 54(3)EPC, relates to the nuclear protein bridging factor TIF-2 and the detection of agonists and antagonists thereto. It discloses a TIF-2 fragment having residues 624-1131 which accidentally contains 4 signature motifs; this fragment is disclaimed. The LXXLL motif mentioned in WO 98/02455 was not present in its priority document.

The present invention is based on the discovery that a short signature motif present in the nuclear proteins is necessary and sufficient to mediate their binding to liganded NRs.

According to one aspect of the present invention there is provided a method for identifying inhibitor compounds capable of reducing the interaction between:
a) a first region which is a signature motif on a nuclear protein, and
b) a second region which is that part of a nuclear receptor which is capable of interacting with the nuclear protein through binding to the signature motif,
wherein:
the nuclear protein is a bridging factor that is responsible for the interaction between a liganded nuclear receptor and a transcription initiation complex involved in regulation of gene expression;
the nuclear receptor is a transcription factor;
the signature motif is B¹XXLL in which B¹ is any natural hydrophobic amino acid, L is leucine and X independently represents any natural amino acid which is the key structural element of a nuclear protein which binds to a liganded nuclear receptor as part of the process of the activation or repression of target genes; and
in which the method comprises taking:
i) the potential inhibitor compound;
ii) the liganded nuclear receptor or a fragment thereof in which the fragment comprises the second region defined in this claim in b) above;
iii) a nuclear protein fragment comprising a signature motif of the nuclear protein with the proviso that a fragment which at least includes residues 624-1131 of TIF-2 is excluded; and
iv) detecting the presence or absence of inhibition of the interaction between ii) and iii).

The term "nuclear protein" means the bridging factors (including coactivators) that are responsible for the interaction between a liganded nuclear receptor and the transcription initiation complex involved in regulation of gene expression (reviewed for steroid hormone receptors in Beato, M., Herrlich, P. & Schutz, G. *Cell* **83**, 851-857 (1995)). The term bridging factor may include part of the transcription initiation complex itself. The term "nuclear receptor" means the family of nuclear receptors such as described in Mangelsdorf, D.J., *et al. Cell* **83,** 835-839 (1995). The term "signature motif" means a short sequence of generally at least about 5 amino acids, preferably 4-10, more preferably 5-10 amino acid residues which is the key structural element of a nuclear protein which binds to a liganded nuclear receptor as part of the process of the activation or repression of target genes. The term "liganded nuclear receptor" means an activated nuclear receptor, for example, with ligand bound thereto. The ligand can take various forms such as for example a hormone, a small molecule compound or a peptide. For example, in the case of some of the nuclear receptors (e.g. PPAR) there are non hormonal peptide ligands e.g. leukotrienes. Although nuclear receptors are generally activated through ligand binding, some receptors, such as for example orphan receptors, may be active without the need for ligand and/ or activated through non-ligand dependent pathways and these receptors are also within the scope of the invention as a less preferred embodiment.

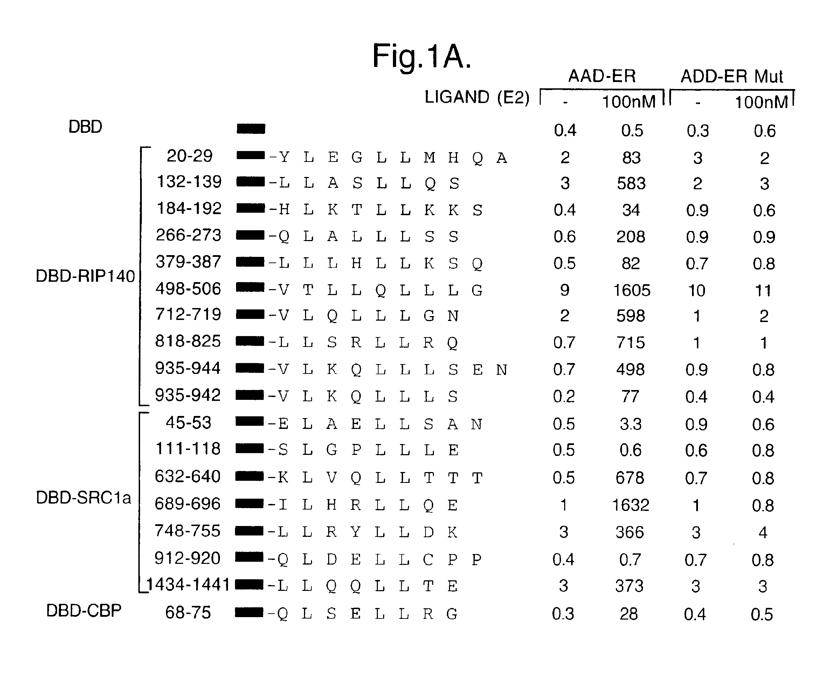

The term "fragment" means an incomplete part. Before the present invention, a skilled person could not have known which fragment or fragments of a nuclear protein could be taken to retain activity. Use of fragments compared with whole proteins is particularly advantageous in screening assays. In a preferred embodiment a preferred fragment size of a nuclear protein is 8-10 amino acids, such as, for example, shown in Figure 1A herein. The liganded nuclear receptor is preferably in the form of a fragment. In general, fragments comprise at least 8 amino acids.

Preferably the signature motif is represented by B¹XXLL in which B¹ is any natural hydrophobic amino acid, L is leucine and X represents any natural amino acid. Values for X within the signature motif are independently selected i.e. X may be the same or different. Preferably B¹ is leucine or valine with leucine being most preferred. In some instances the preferred signature motif is further defined as B²B¹XXLL wherein "B²" is a hydrophobic amino acid residue as defined for B¹. A "natural hydrophobic amino acid" is defined as any one of isoleucine, leucine, methionine, phenylalanine, tryptophan, tyrosine or valine. Preferably the signature motif is in the conformation of a helix, preferably an amphipathic helix, and the leucine residues form a hydrophobic face thereof. Preferably the signature motif is positioned within a molecule so that it is available at the surface thereof for interaction with proteins. Preferably values of X do not include Cys or Pro. Preferably at least one value of X is not a natural hydrophobic amino acid or proline. One value of X is preferably independently selected from Arg, Asn, Asp. Glu, Gln, His, Lys, Ser, Thr, Gly or Ala, more preferably one value of X is independently selected from Arg, Asn, Asp, Glu, Gln, His or Lys. Without wishing to be bound by theoretical considerations, it is believed that preferred values of X favour the signature motif forming an amphipathic helix.

After the first filing date of the present invention there was a simultaneous publication of the identification of LXXLL motifs by two groups (one of which included the inventors of the present invention), namely Heery *et al.* Nature, 12^{th} June 1997, 387, 733-736 & Torchia *et al,* Nature. 12^{th} June 1997, 387, 677-684.

Herein we show that the ability of a nuclear protein (SRC 1) to bind a nuclear receptor (liganded ER) and enhance its transcriptional activity is dependent upon the integrity within the nuclear protein of the signature motif (LXXLL; SEQ ID NO: 1), as well as key hydrophobic residues in the conserved helix (Helix 12) of NRs required for their ligand-induced activation function (AF-2) ¹⁴. The signature motif is also found in TIF1, TIF2, p300. RIP 140 and the TRIP proteins, and occurs within regions of these proteins known to be sufficient for interaction with NRs. Thus the LXXLL motif (SEQ ID NO: 1) is a signature sequence which facilitates the interaction of diverse proteins with nuclear receptors, and thus is a key part of a new family of nuclear proteins.

A preferred nuclear protein is a coactivator, in particular the nuclear protein includes any one of RIP 140. SRC-1, TIF2, CBP, p300, TIF1, Trip1, Trip2, Trip3, Trip4, Trip5, Trip8 or Trip9. Further preferred nuclear proteins include p/CIP, ARA70 & Trip230.

In this specification a reference to a nuclear protein or nuclear receptor includes isoforms thereof unless stated or otherwise implicit from the context. An isoform is one of a family or collection of related proteins derived from a single gene. Thus isoforms may differ slightly in their amino acid sequences such as for example from differential splicing of exons following transcription. SRC1a is an example of an isoform of SRC1. Two isoforms of SRC-1, namely SRC1a and SRC1e, have been shown to contain differences in number of signature motifs and to be functionally distinct in so far as they appear to play different roles in ER-mediated transcription (Kalkhoven *et al,* 1998, EMBO Journal, 17, 232-243).

Nuclear receptors are transcription factors. A preferred transcription factor comprises at least part of a conserved amphipathic α-helix, and especially preferred is retinoic acid receptor or a steroid hormone receptor. Preferred steroid hormone receptors are oestrogen receptor, progesterone receptor, androgen receptor and glucocorticoid receptor with oestrogen receptor being especially preferred.

Preferably the second region comprises at least part of a conserved amphipathic α-helix such as for example Helix 12 in the oestrogen receptor which is especially preferred.

An especially preferred combination of nuclear receptor and nuclear protein is one in which the nuclear receptor is oestrogen receptor and the nuclear protein is selected from SRC1, TIF2, CBP and p300, with SRC1 and especially SRC1a being most preferred.

A preferred method is in the form of a 2-hybrid assay system. Such assay systems are well known in the art; suitable references include Fields & Stemglanz (1994) TIG, August 1994, 10, 286-292 and US patent 5283173.

Any suitable assay design may be employed such as for example radioisotopic assay, scintillation proximity assay (reviewed by ND Cook, 1996, Drug Discovery Today, 1, 287-294) or fluorescence, particularly time resolved fluorescence, assay (reviewed by MV Rogers, 1997, Drug Discovery Today, 2, 156-160). High-throughput screeening technologies have been reviewed by Houston & Banks in Current Opinion in Biotechnology 1997, 8, 734-740.

In a preferred embodiment of the invention the potential inhibitor is a member of a peptide library based on a signature motif.

Encoded peptide libraries generally have a maximum of 20 possible amino acids at any one position. In practice, using current technology, it is difficult to screen libraries of more than 10⁷- 10⁸ members, which means that it is difficult to randomise more than 6 positions in a peptide. Hence, narrowing down the nuclear protein binding region to a signal motif is of great advantage if a peptide library approach is to be employed.

A peptide library is a collection of peptides of varying sequences. There are in general two ways to generate peptide libraries (reviewed by Scott, 1992; Birnbaum and Mosbach, 1992; Houghten, 1993;see also Abelson, 1996). The first approach is to generate libraries in which positive peptides are identified through the sequencing of the peptides themselves. Mixtures of peptides may be chemically synthesised in such a way that the peptides are linked to beads, so that each bead contains only one peptide. If a bead is identified which contains a positive peptide, the bead may be recovered and the peptide identified by chemical sequencing. This approach was first demonstrated using the ability of antibodies to identify specific six amino acid peptides from mixtures (Lam *et al,* 1991). The importance of using beads is that the identification event (in this case the antibody:peptide interaction) leads to the recovery of a bead which contains more peptide than that bound by the antibody itself. In a related approach mixtures of free peptides can be synthesised and screened in pools: by using a deconvolution process, positive peptides are identified (Houghten *et al,* 1991).

The second approach is to generate libraries in which positive peptides are identified by sequencing a molecule which is associated in some way with the peptide. Peptides and some other molecule, such as a nucleic acid, can be cosynthesised on beads, so that each nucleic acid "tags" the peptide found on the same bead. If a bead is identified which is positive, sequencing the nucleic acid will identify the peptide on this bead (Brenner and Lerner, 1992). Alternatively, peptide libraries can be generated using the gene expression machinery of living organisms. In this approach, it is not necessary to make a library of peptide molecules. Instead a library of DNA molecules is constructed, so that each molecule encodes a peptide with a different sequence. This encoded library must then be expressed in a suitable host organism in order that the peptide library be produced. The library is then screened. It is essential that the nucleic acid which encodes the library remain physically linked to the protein in some way, so that recovery, or identification, of the active peptides leads to recovery of the DNA which encodes these peptides. The sequences of the active peptides may then be deduced by sequencing of the DNA which encodes them. Several variations of this approach have been described.

The mostly widely used version of this approach is to express peptides as part of the coat proteins of a virus such as M13. The viruses can be screened by the ability of this coat protein to bind target proteins (such as antibodies (Devlin *et al,* 1990; Scott and Smith, 1990; Cwirla et al, 1990) or receptors (the atrial natriuretic peptide receptor: Cunningham *et al* (1994) and the thrombopoietin receptor (Cwirla *et al,* 1997). The approach may also be used to find protease inhibitors through their ability to bind to proteases (Roberts *et al,* 1992; Markland *et al,* 1996) as well as to find optimal substrates for proteases, such as stromelysin and matrilysin (Smith *et al,* 1995) and subtilisin (Matthews and Wells, 1993)

An intracellular approach to the generation of peptides that recognise certain proteins is to use the yeast two-hybrid system. In the two-hybrid system, interacting proteins are fused to domains of transcription factors. If a protein:protein interaction occurs, then transcription of a reporter gene is stimulated (Fields and Song, 1989). By making one component of the two hybrid system a peptide library, and selecting for cells in which reporter gene output occurs, it is possible to isolate peptides which bind to a target protein, this approach was used to identify peptides which bound to the retinoblastoma protein (Yang *et al,* 1995). In a similar approach, Colas *et al* (1996) expressed the peptide library as a loop in the surface of the E. coli TrxA protein and isolated peptides which bound to cyclin-dependent kinase 2 (Cdk2).

According to another aspect of the present invention there is provided a method of reducing the interaction between
a) a first region which is a signature motif on a nuclear protein, and
b) a second region which is that part of a nuclear receptor which is capable of interacting with the nuclear protein through binding to the signature motif,
in which the method comprises adding an inhibitor in the presence of the nuclear receptor and the nuclear protein, the inhibitor being characterised in that it reduces the interaction between the first region of the nuclear protein and the second region of the nuclear receptor.

According to another aspect of the present invention there is provided a peptide selected from any one of the following peptides; PQAQQKSLLQQLLT (SEQ ID NO: 2), KLVQLLTTT (SEQ ID NO: 3), ILHRLLQE (SEQ ID NO: 4), or LLQQLLTE (SEQ ID NO: 5). Peptides may be prepared using conventional techniques for example using solid phase synthesis and Fmoc chemistry. These peptides are expected to be useful in the treatment of oestrogen responsive tumours. Inhibitors of the invention are expected to be useful in the treatment of any disease mediated through interaction between a signature motif on a nuclear protein and a nuclear receptor. For example, suitable inhibitors are expected to be useful in treatment of cancer or inflammation.

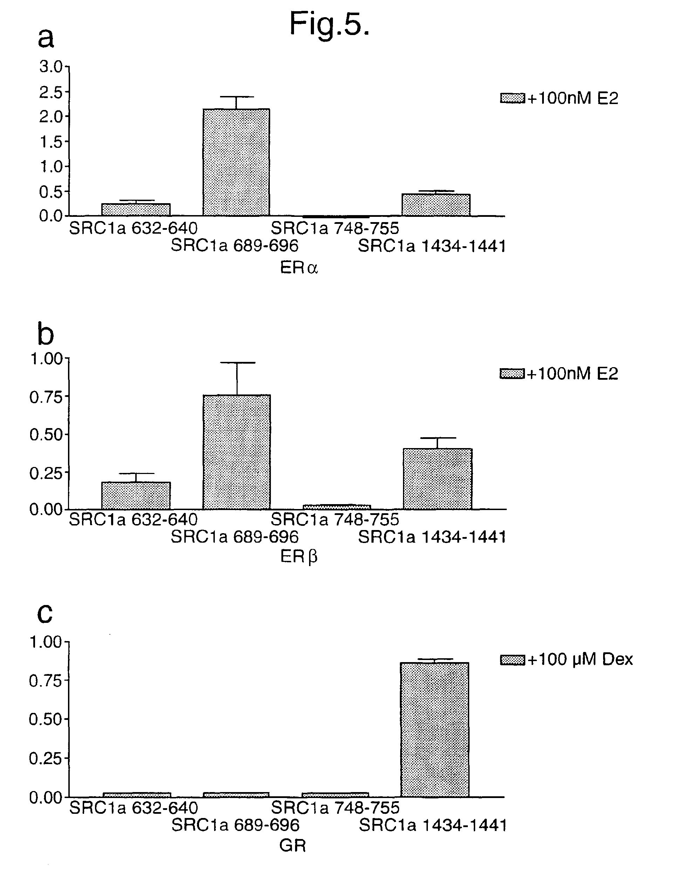

Whilst the signature motif is demonstrated herein to apply across nuclear proteins as a class it is expected that different nuclear receptors display both coactivator and signature motif preferences that contribute to specificity of hormonal response (Ding *et al,* 1998, Molecular Endocrinology, 12, 302-313) which in turn points to selective pharmaceutical intervention opportunities. Figures 1A and 5 below also indicate that individual motifs may differ in the strength to which they bind to nuclear receptors.

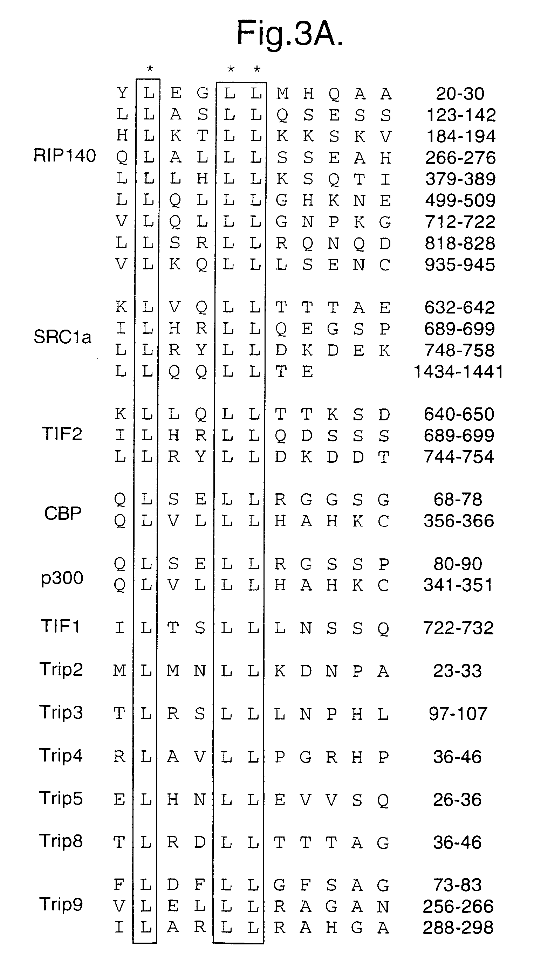

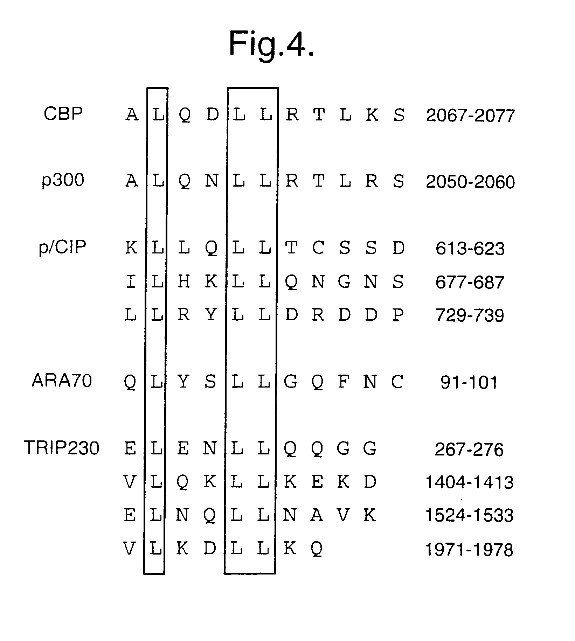

Note the NR box of Le Douarin *et al* (discussed above) did not disclose a signature motif within the meaning of the present invention because, for example, the NR box within the meaning of Le Douarin would be present in at most only 4 of the 39 signature motifs identified by the present invention in Figures 3A & 4 (see below). Furthermore. Le Douarin *et al* did not even suggest inhibitors of nuclear receptor - nuclear protein interaction.

The term "antibodies" is meant to include polyclonal antibodies, monoclonal antibodies, and the various types of antibody constructs such as for example F(ab')₂, Fab and single chain Fv. Antibodies are defined to be specifically binding if they bind with a Kₐ of greater than or equal to about 10⁷ M⁻¹. Affinity of binding can be determined using conventional techniques, for example those described by Scatchard *et al., Ann. N. Y Acad. Sci.,* 51: 660 (1949).

Polyclonal antibodies can be readily generated from a variety of sources, for example, horses, cows, goats, sheep, dogs, chickens, rabbits, mice or rats, using procedures that are well-known in the art. In general, immunogen is administered to the host animal typically through parenteral injection. The immunogenicity may be enhanced through the use of an adjuvant, for example, Freund's complete or incomplete adjuvant. Following booster immunizations, small samples of serum are collected and tested for reactivity. Examples of various assays useful for such determination include those described in: *Antibodies: A Laboratory Manual,* Harlow and Lane (eds.), Cold Spring Harbor Laboratory Press, 1988; as well as procedures such as countercurrent immuno-electrophoresis (CIEP), radioimmunoassay, radioimmunoprecipitation, enzyme-linked immuno-sorbent assays (ELISA), dot blot assays, and sandwich assays, see U.S. Patent Nos. 4,376,110 and 4,486,530.

Monoclonal antibodies may be readily prepared using well-known procedures, see for example, the procedures described in U.S. Patent Nos. 4,902,614, 4,543,439 and 4,411,993; Monoclonal Antibodies, Hybridomas: *A New Dimension in Biological Analyses,* Plenum Press, Kennett, McKearn, and Bechtol (eds.), (1980).

Monoclonal antibodies can be produced using alternative techniques, such as those described by Alting-Mees *et al.,* "Monoclonal Antibody Expression Libraries: A Rapid Alternative to Hybridomas", *Strategies in Molecular Biology* 3: 1-9 (1990) which is incorporated herein by reference. Similarly, binding partners can be constructed using recombinant DNA techniques to incorporate the variable regions of a gene that encodes a specific binding antibody. Such a technique is described in Larrick *et al., Biotechnology, 7* : 394 (1989).

According to a further feature of the invention there is provided a pharmaceutical composition which comprises a peptide as defined above, or a pharmaceutically-acceptable salt thereof, in association with a pharmaceutically-acceptable diluent or carrier.

The composition may be in a form suitable for oral use, for example a tablet, capsule, aqueous or oily solution, suspension or emulsion; for topical use, for example a cream, ointment, gel or aqueous or oily solution or suspension; for nasal use, for example a snuff, nasal spray or nasal drops; for vaginal or rectal use, for example a suppository; for administration by inhalation, for example as a finely divided powder such as a dry powder, a microcrystalline form or a liquid aerosol; for sub-lingual or buccal use, for example a tablet or capsule; or for parenteral use (including intravenous, subcutaneous, intramuscular, intravascular or infusion), for example a sterile aqueous or oily solution or suspension. In general the above compositions may be prepared in a conventional manner using conventional excipients. For peptidic inhibitors, parenteral compositions are preferred.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 2 g of active agent compounded with an appropriate and convenient amount of excipients which may vary from about 5 to about 98 percent by weight of the total composition. Dosage unit forms will generally contain about 1 mg to about 500 mg of an active ingredient.

The invention is illustrated by the non-limiting Examples below in which, unless stated otherwise: temperatures are expressed in degrees Celsius; and peptide sequences are listed N-terminus to C-terminus.

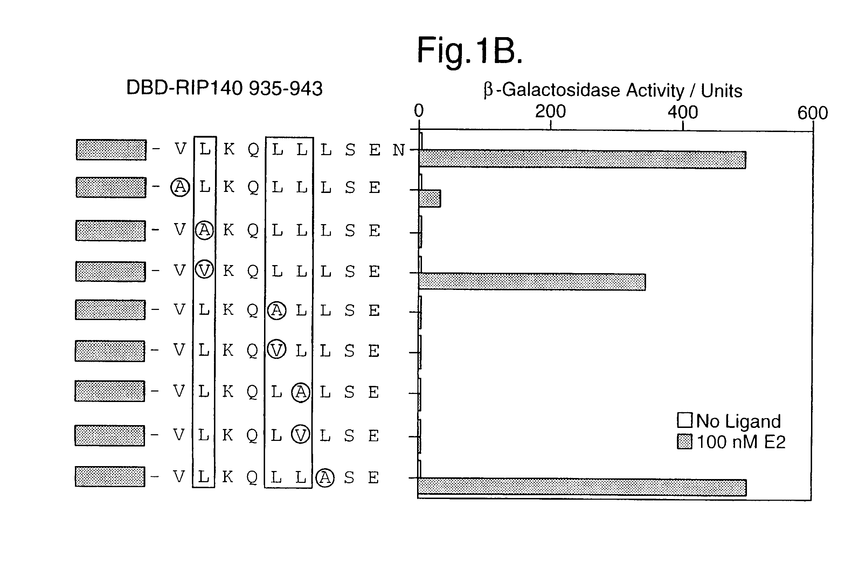

**Figures 1a/1b show the interaction of LXXLL motifs derived from coactivators with the ER.**
   Figure 1a: Yeast two hybrid interactions of LXXLL motifs, derived from the proteins RIP140, SRC1a and CBP with the LBDs of wild type or mutant ER. The sequences of the LXXLL motifs in the DNA binding domain (DBD) fusion proteins are indicated. DBD-LXXLL proteins were coexpressed with AAD-ER or AAD-ER Mut, which consist of an acidic activation domain (AAD) fused to the LBD of the wild-type ER, or a transcriptionally defective ER mutant, respectively. Reporter activities were determined in the presence or absence of 10⁻⁷M 17-β-estradiol (E2) and expressed as units of β-galactosidase activity. Sequences listed in Figure 1a, from top to bottom, are listed as SEQ ID NO: 6-23 respectively.
   Figure 1b: Effects of mutations in the RIP140 LXXLL motif located at amino acids 935-943 on binding of AAD-ER. Conserved leucine residues are boxed and mutated residues are circled. The reporter activity was determined in the presence (black bars) or absence (white bars) of 10⁻⁷ M E2. Sequences listed in Figure 1b, from top to bottom, are listed as SEQ ID NO: 24-32 respectively.

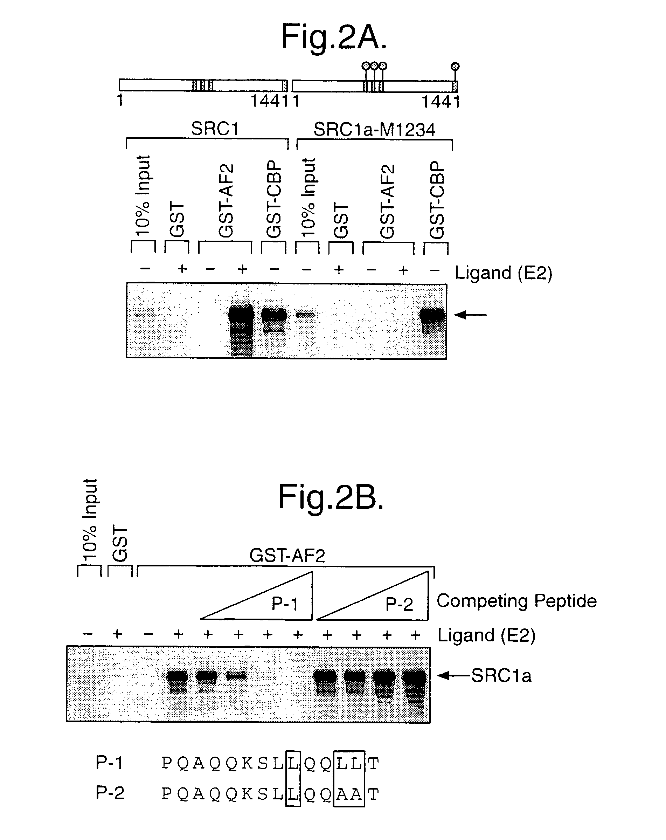

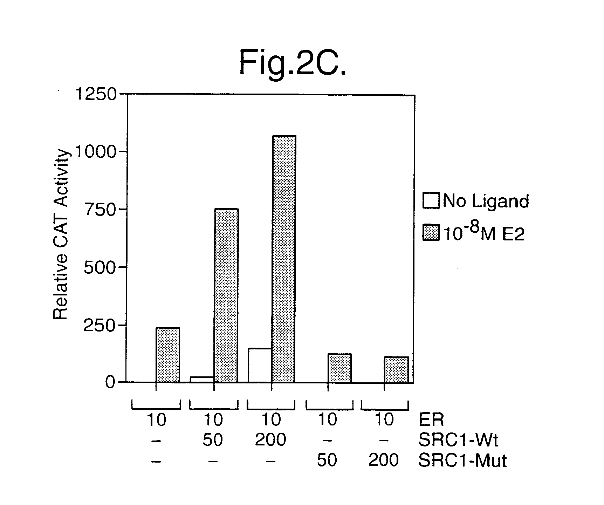

**Figures 2a/2b/2c show that LXXLL motifs are required for binding of SRC1 to the ER LBD *in vitro* and for the ability of SRC1 to enhance ER activity *in vivo.***
   Figure 2a: Wild type (SRC1) and mutant (SRC1a-M1234) SRC1 proteins are shown schematically. The black bars represent the approximate locations of the LXXLL binding motifs in the linear SRC1a sequence and the shaded circles indicate the mutation of LXXLL binding motifs by replacement of conserved leucine residues with alanines (see Methods). Binding of wild type SRC1a or SRC1a-M1234 mutant to glutathione S transferase (GST) alone, to the ligand binding domain (aa 313-599) of ER (GST-AF2), or the SRC1 binding domain (aa 2058-2163) of CBP (GST-CBP) in the presence (+) and absence (-) of 10⁻⁶M E2. The signals obtained with 10% of the input of [³⁵S] -labelled wild type and mutant SRC1 proteins are shown.
   Figure 2b: The ability of increasing amounts of the peptides P-1 (SEQ ID NO: 2) and P-2 (SEQ ID NO: 72) to compete against the binding of wild type SRC1a to GST-AF2 in the presence of ligand is shown. The sequences of the P-1 and P-2 peptides are given at the foot of Fig 2b, and the conserved leucines and alanine substitutions are boxed.
   Figure 2c: Wild type but not mutant SRC1e M123 potentiates activation by ER of the reporter gene 2ERE-pS2-CAT in transiently transfected Hela cells. Reporter activities obtained from extracts of transfected cells grown in the absence (white columns) or presence (black columns) of ligand (10⁻⁸M E2). The amounts of ER, SRC1-wt and and SRC1-mut expression plasmids used in the transfections are indicated below the graph. The activities shown are averaged from duplicates.

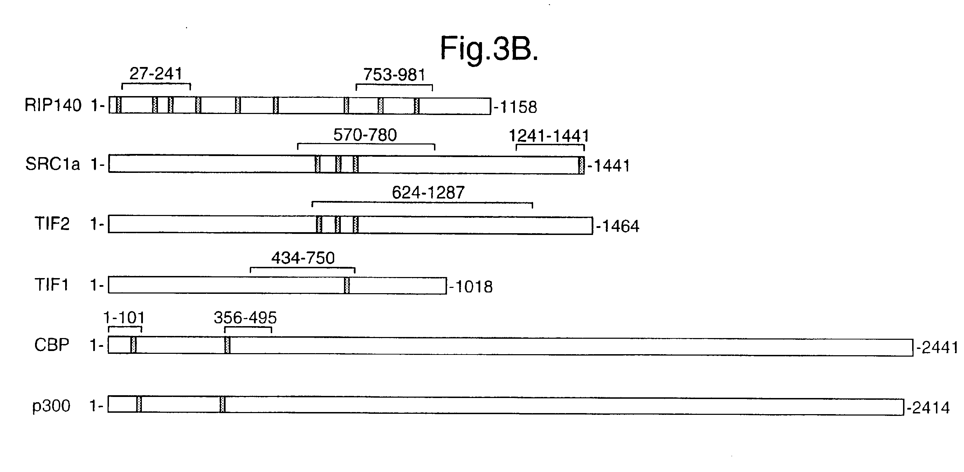

**Figures 3a/3b & 4 show that the LXXLL sequence is a signature motif in proteins that bind the LBDs of NRs.**
   Figure 3a: Alignment of LXXLL motif sequences present in human RIP140¹⁰. human SRC1a, mouse TIF2 6, mouse CBP ^{23.24}, p300 ³³, mouse TIF1¹¹ and human TRIP proteins 12. The conserved leucines are boxed and the amino acid numbers are given for each motif. Sequences listed in Figure 3A, from top to bottom, are listed as SEQ ID NO: 33-61 respectively.
   Figure 3b: Schematic representation of the incidence of LXXLL motifs (black bars) in the sequences of proteins which bind NRs. The amino acid boundaries of the known NR binding sites are also shown.
   Figure 4: Alignment of LXXLL motif sequences present in CBP, P300, p/CIP, ARA70 & TRIP 230. The conserved leucines are boxed and the amino acid numbers are given for each motif. Sequences listed in Figure 4, from top to bottom, are listed as SEQ ID NO: 62-71 respectively.
**Figure 5 shows that the precise nature of the LXXLL signature motif affects the strength of the interaction between SRC-1a and the LBDs of ERα, ERβ and GR.**
   Figure 5a: The ERα LBD binds to the 4 signature motifs of SRC-1a with differing affinities in yeast 2-hybrid assays (SRC-1a motif 1-4 = SEQ ID 73-76). The order (decreasing affinity) is SRC-1a motif 2> SRC-1a motif 4 > SRC-1a motif 1> SRC-1a motif 3.
   Figure 5b: The ERβ LBD binds to the signature motifs of SRC-1a with the same relative affinities seen with ERα. The order (decreasing affinity) is SRC-1a motif 2 > SRC-1a motif 4> SRC-1a motif 1 > SRC-1a motif 3.
   Figure 5c: The GR LBD binds to the signature motifs of SRC-1 with differing affinities and 5 the rank order of affinities differs to those seen with ERα and ERβ. The order (decreasing affinity) is SRC-α motif 4 > SRC-1a motif 1 = SRC-1a motif 2 = SRC-1a motif 3.
In Figure 5 the y-axis units represent relative β-galactosidase activity. In Figure 5 the x-axis motif numbering only indicates part of the actual sequence used which was as follows: 627-640. 684-696, 743-755 and 1428-1441.

The following abbreviations are used.

| | |
|---|---|
| AAD | acidic activation domain |
| AF | activator function |
| DBD | DNA binding domain |
| E2 | 17-β-estradiol |
| ER GR | estrogen receptor glucocorticoid receptor |
| GST | glutathione S transferase |
| LBD | ligand binding domain |
| NR | nuclear receptor |
| PCR | polymerase chain reaction |
| RAR | retinoic acid receptor |
| RIP | receptor interacting protein |
| RXR | retinoid X receptor |
| SRC | steroid receptor coactivator |
| TIF | transcriptional intermediary factor |
| TRβ | thyroid hormone receptor β |

Standard amino acid abbreviations have been used.

| | | |
|---|---|---|
| Alanine | Ala | A |
| Arginine | Arg | R |
| Asparagine | Asn | N |
| Aspartic Acid | Asp | D |
| Cysteine | Cys | C |
| Glutamic Acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any amino acid | Xaa | X |

Point mutations will be referred to as follows: natural amino acid (using the 1 letter nomenclature) , position, new amino acid. For example "L636A" means that at position 636 a leucine (L) has been changed to alanine (A). Multiple mutations will be shown between square brackets.

### Example 1

### Mapping of interaction sites between Nuclear Receptor and Nuclear Protein

It has been previously demonstrated that the 140 kDa receptor interacting protein (RIP140) bound directly to NRs through at least two distinct sites located at the N- and C-termini of the protein ²¹. To map these interaction sites in more detail, we examined a series of twenty different PCR-generated fragments of RIP140 coding sequence fused in frame with a heterologous DBD. for interaction with NRs in a two hybrid system. Remarkably, although the different constructs spanned the entire 1158 amino acids of RIP 140 sequence, all but two displayed ligand-dependent interaction with ER, including five non-overlapping RIP140 sequences. By comparison of the sequences of the shortest interacting fragments we identified a short motif (LXXLL) common to all interacting fragments. In total, nine copies of the motif were identified in the RIP 140 sequence, but the motif was absent in fragments showing no binding activity in our experiments.

To determine if these short sequences were sufficient to bind to NRs, we constructed a series of proteins consisting of a DBD fused to eight to ten amino acids incorporating one copy of each of the nine LXXLL motifs. As shown in **Fig**. 1a, each of the nine motifs present in RIP140 displayed strong ligand-dependent interaction with the LBD of the ER whereas the DBD alone showed no ability to bind. (Note that of the 10 motifs listed for RIP140, the 10^{th} is a repeat of the 9^{th} locus). Comparable results were obtained with the LBD of RAR (data not shown). Mutation of hydrophobic residues within H12 abolish AF2 activity and prevent the recruitment of RIP140 ¹⁰, TIF 1¹¹, TIF2 6, SUG 1 ¹³ and SRC1. Similarly, mutation of H12 residues M543 and L544 in the ER abolished the ligand-dependent interaction of all nine LXXLL motifs with ER (**Fig**. 1a). Taking these results together, we conclude that a short conserved motif comprised within as little as eight amino acids is sufficient to bind to transcriptionally active NRs. This discovery that such a relatively small motif can affect the interaction between two relatively large molecules is unprecedented in this field.

Secondary structure analysis using the Phd program ²² revealed that each of the nine copies of this motif in RIP140 occurred within a region predicted to be α-helical in nature, in which the conserved leucines would form a hydrophobic face.

### Example 2

### Mutational Analysis of a Signature Motif

To determine the sequence constraints required to observe a functional interaction, we carried out a partial mutational analysis of one of the RIP140 motifs (amino acids 935-943; **Fig.**1b). While western blot analysis showed no significant variation in the expression of the wild type and mutant fusion proteins (data not shown), mutation of valine 935 to alanine resulted in approximately ten fold reduction in the reporter activity in the presence of ligand which, when coupled with the observation that the first amino acid is hydrophobic in seven of the nine LXXLL motifs in RIP140. may indicate a preference for a hydrophobic residue at this position. Strikingly, mutation of any one of the three conserved leucine residues L936, L939 or L940 to alanine resulted in a complete loss of binding to the LBD of ER (**Fig**. 1b) and RAR (data not shown), emphasising their importance in mediating the interaction with NRs. In contrast, mutation to alanine of L941 (which is not conserved among the motifs; see **Fig**. 3a), had no effect on the ability of this sequence to bind to the ER LBD. Replacement of a conserved leucine residue with a valine was tolerated at L936, but not at L939 or L940 indicating that hydrophobic character alone is not sufficient to maintain an interaction with ER (Fig. 1b). The amino acids K937, Q938, S942 and E943 were not subjected to mutagenesis as they are not conserved among the motifs we have identified (see Fig. 3a).

### Example 3

### Analysis of Signature Motifs in Nuclear Proteins

The steroid receptor coactivator SRC1, which stimulates ligand-dependent transcriptional activity, was originally identified as a partial cDNA encoding a protein capable of interacting with the progesterone receptor by means of a 196 amino acid C-terminal region ³. We noted that the eight most C-terminal amino acids fit the LXXLL consensus, and indeed this sequence (DBD-SRC1a 1434-1441) displayed strong ligand-induced binding to ER, but not the ER H12 mutant (Fig. 1a). Subsequent studies have identified full-length SRC1 (SRC1a) from mouse (1459 amino acids) ^{4,5} and human (1441 amino acids) tissues. Both murine and human SRC I a proteins interact with multiple NRs, and contain an additional interaction region between residues 569 to 789 ⁵ and 570-780, respectively. Three copies of the LXXLL motif were identified in this central interaction domain of human SRC1a (see Fig. 3a & 3b), each of which displayed ligand-dependent binding to both ER (Fig. 1a) and RAR (data not shown) in the two hybrid assay, but not the ER H12 mutant. Interestingly, the sequences and relative positions of the three motifs in the central domain of SRC1a are conserved in the related coactivator protein Transcriptional Intermediary Factor 2 (TIF2) (Fig. 3a + b), and correspond to the region of TIF2 known to bind to NRs⁶. However, unlike SRC1a, TIF2 appears to lack a motif at its C-terminus. In addition, we noted that SRC1 contains three other sequences matching the LXXLL consensus. Although the motif at residues 45-53 is predicted by the Phd program to be α-helical and lies within the basic helix-loop-helix domain at the N-terminus of SRC1a⁵, it showed only a very weak (6-fold) interaction with liganded ER (Fig. 1a) or RAR (not shown) in the yeast two hybrid assay. This is consistent with the observed absence of strong NR-binding activity associated with the N-terminus of SRC1. The other two motifs within residues 111-118 and 912-920 both contain proline residues and are unlikely to adopt α-helical structure according to the Phd program. Indeed, these sequences showed no detectable interaction with NRs in our binding assays (Fig. 1a), which strongly suggests a preference for appropriate secondary structure for binding of LXXLL sequences to NRs.

Recent reports have indicated that CBP/p300 proteins, which were originally identified as coactivators for CREB ^{23,24}, are coactivators for many transcription factors including NRs ^{4,5,8,9} and may serve as integrators of several signalling pathways ⁴. CBP was shown to bind directly to NRs via its N-terminal 101 amino acids ⁴ , with a possible RXR-specific binding site between residues 356-495 ⁸. Our analysis showed that the CBP sequence harbours copies of the LXXLL motif within positions 68-78, and 356-364, which are conserved in the p300 sequence (amino acids 80-90 and 341-351; Fig 3a). Indeed, when tested in the two hybrid assay, the N-terminus of CBP (amino acids 1-101; data shown) and the LXXLL motif at residues 68-75 of the CBP sequence (Fig. 1a) displayed ligand-dependent binding to ER, but not the transciptionally defective ER mutant (Fig. 1a).

### Example 4

### The Binding Of Coactivator Proteins To NRs Is Dependent On Signature Motifs

To demonstrate that the binding of coactivator proteins to NRs is dependent on LXXLL motifs, we introduced alanine substitutions in SRCla at the conserved leucine couplets at residues [L636A, L637A, L693A, L694A, L752A, L753A, L1438A, L1439A] thus effectively creating a mutant protein (SRC1a-M 1234) in which all the four functional binding motifs were disabled. We then compared the ability of *in vitro* translated SRC1a and SRC1a-M1234 to bind to the ligand binding domain of the mouse estrogen receptor fused to glutathione-S-transferase (GST-AF2) in GST pulldown experiments (Maniatis *et al.,(* 1982) Molecular Cloning. A Laboratory Manual. Cold Spring Harbour Laboratory, Cold Spring Harbour, New York.). As shown in Fig. 2a, while wild type SRC1a protein displayed ligand-dependent binding to GST-AF2, SRC1a-M1234 failed to bind to GST-AF2 either in the presence or absence of ligand. To confirm that the mutations did not induce gross structural disruption of the SRC1a-M1234, we compared the ability of the *in vitro* translated proteins to interact with amino acids 2058-2163 of CBP, which was previously defined as the SRC1 binding domain ⁴. Both proteins retained strong binding to GST-CBP (Fig. 2a) indicating that this SRC1 function remained intact in both wild type and mutant proteins. In addition we showed that the binding of wild type SRC1a to GST-AF2 was competed by increasing concentrations of a short peptide (P1) corresponding to the motif at the C-terminus of SRC1a (Fig. 2b). In contrast, a similar peptide (P2) in which the LXXLL motif was mutated, or peptides unrelated to the LXXLL motif (data not shown), did not compete the binding of SRC1a to GST-AF2 (Fig. 2b).

Finally, to demonstrate that LXXLL motifs are necessary for the function of SRC1 *in vivo,* we compared the abilities of wild type SRC1 and a mutant protein in which all LXXLL motifs were disabled to enhance the activity of mouse ER in transient transfection experiments. As shown in Fig. 2c, wild type SRC1 enhanced the activity of ER in a concentration-dependent manner. In contrast, the SRC1 mutant, which was unable to bind ER (Fig. 2a) had no stimulatory effect, but reduced ER activity by up to 50% at the highest concentration (Fig. 2c). This apparent dominant negative property of the mutant SRC1 is likely due to its ability to maintain interactions with CBP while failing to interact with NRs (Fig. 2a). This result is of interest given the recent evidence that SRC1 and CBP/p300 may exist as a complex *in vivo* ⁹, and that CBP also has NR binding activity ^{4, 8}, as our data suggest that the interactions between NRs and CBP are insufficient to compensate for the inability of the SRC1a mutant protein to bind NRs, at least under these conditions. It remains to be determined whether NRs are engaged simultaneously by p160 and p300 proteins functioning independently or as a complex.

Examination of the sequences of other proteins known to bind to NRs revealed them to contain one or more copies of the LXXLL motif. TIF1 contains a single motif (residues 722-732) within the minimal region known to be required for its interaction with NRs^{11.25} The truncated proteins TRIPs2-5, TRIP8 and TRIP9, which were isolated in a two hybrid screen for TR-interacting proteins ¹², each contain at least one copy of the LXXLL motif (Fig. 3a), whereas the motif was absent in TRIPs whose interaction with TR was ligand-independent. An alignment of a selection of these sequences is shown in Fig. 3a, while Fig. 3b shows the incidence of motifs in the sequences of RIP140, SRC1a, TIF1, TIF2, CBP and p300, and the boundaries of known receptor interaction domains in these proteins. Interestingly, motifs were also identified in several other proteins for which evidence exists of interaction with NRs, including Ara70 ²⁶, SW13 ²⁷, and the RelA (p65) subunit of NFκ-B ²⁸, although the receptor interaction domains in these proteins have not been mapped. The ability of other proteins containing LXXLL motifs to bind to NRs will depend on their subcellular localisation, as well as the α-helicity and surface accessibility of the motifs. While it is clear that the conserved leucine residues are essential for the function of the motif, other amino acids may also be important given the degree of sequence conservation of equivalent motifs in SRC1/TIF2 or CBP/p300.

As many NR binding proteins contain multiple copies of the LXXLL motif it remains to be established whether this facilitates the simultaneous contact of individual partners in homo- and heterodimers of NRs, or whether it serves to provide alternative interaction surfaces to accomodate conformational changes imposed by the binding of NRs to different response elements. The systematic mutation of LXXLL motifs in coactivators such as SRC1 and CBP may allow us to decouple crosstalk or synergy between different signal transduction pathways, and thus provide a better understanding of their proposed roles as coactivators and integrators.

### Example 5

### Two Hybrid Interaction Assays

The yeast reporter strain used for all two hybrid assays was W303-1B (HMLα MATα HMRa his3-11, 15 trpl-1 ade2-1 can1-100 leu2-3, 11, ura3) carrying the plasmid pRLΔ21 - U3ERE which contains a lacZ reporter gene driven by three estrogen response elements (EREs) ²⁹. The plasmids pBL1 and pASV3 which express the human ER DNA binding domain (DBD) and the VP16 acidic activation domain (AAD) respectively ³⁰, were used to generate DBD or AAD fusion proteins for two hybrid interaction analyses. DBD-LXXLL motif fusion proteins were generated by ligation of phosphorylated, annealed oligonucleotide pairs into the pBL1 vector. AAD-ER was constructed by cloning a PCR fragment encoding amino acids 282-595 of the human ER into pASV3. AAD-ER Mut was constructed in a similar fashion except that the amino acids M543 and L544 or ER were mutated to alanines by recombinant PCR. All fusion constructs were fully sequenced. Transformants containing the desired plasmids were obtained by selection for the appropriate plasmid markers and were grown to late log phase in 15 ml of selective medium (yeast nitrogen base containing 1% glucose and appropriate supplements) in the presence or absence of 10⁻⁷ M 17-β-estradiol (E2).

The expression of DBD- and AAD- fusion proteins in yeast cell-free extracts was verified by immunodetection using a monoclonal antibody recognising the human ER (a gift from P. Chambon, Strasbourg). The antibody recognises the "F" region of the LBD in the human ER, and also the "F" region tag at the N-termini of the DBD fusion proteins ³⁰. Equal amounts of protein were electrophoresed on polyacrylamide gels and transferred to nitrocellulose for western blotting. The preparation of cell-free extracts by the glass bead method and the measurement of β-galactosidase activity in the extracts were performed as previously described ²⁹. Two hybrid experiments were repeated several times, and the data shown in Figs. 1a and 1b represent reporter activities as measured in a single representative experiment. The β-galactosidase activities are expressed as nmoles/minute/µg protein.

### Example 6

### In Vitro Binding And Peptide Inhibition Assays

GST-AF2 consists of the ligand binding domain of the mouse ER (amino acids 313-599) fused to glutathione-S-transferase and has been described previously ³¹. GST-CBP consists of GST fused to the SRC1-binding domain of CBP and was constructed by cloning a PCR fragment encoding residues 2058-2163 of mouse CBP into the vector pGEX2TK (Pharmacia). Human SRC1a and SRC1e cDNAs were isolated form a human B cell cDNA library and cloned into a modified version of the expression vector pSG5. SRC1a M1234 and SRC1eM123 were constructed by recombinant PCR to introduce the mutations [L636A, L637A, L693A, L694A, L752A. L753A, L1438A, L1439A] or [L636A, L637A, L693A, L694A, L752A, L753A] respectively. All SRC1 constructs were fully sequenced. GST-SEPHAROSE^{™} beads were loaded with GST alone or GST-fusion proteins prepared from bacterial cell-free extracts. [³⁵S]-labelled SRC 1 proteins were generated by *in vitro* translation and tested for interaction with GST proteins in the presence or absence of 10⁻⁶M estradiol (E2) as previously described ²¹. Binding was carried out for 3 hours at 4° with gentle mixing in NETN buffer (100 mM NaCl, 1 mM EDTA, 0.5 % NP-40, 20 mM Tris HCl, pH 8.0) containing protease inhibitors in a final volume of 1 ml. Peptides P- 1 and P-2 were dissolved in water at a concentration of 4mg/ml and added individually to GST-binding reactions immediately before the addition of ligand. The increasing amounts of peptide added in the competition experiments shown corresponded to 2.5, 5, 12.5 and 25 µM.

Using analogous methodology, peptides KLVQLLTTT (SEQ ID NO: 3), ILHRLLQE (SEQ ID NO: 4) and LLQQLLTE (SEQ ID NO: 5) can be shown to be inhibitors.

### Example 7

### Transient Reporter Assays

Hela cells were transfected with 1µg of reporter 2ERE-pS2-CAT ³², 150 ng of β-galactosidase expression plasmid (internal control), 10 ng of ER expression plasmid and 50 or 200 ng of SRC1 expression plasmids or empty vector per well (in duplicate) using 24-well plates. Transfected cells were incubated overnight in Dulbecco's modified Eagle's medium without phenol red and containing 10% charcoal-treated FBS, and washed in fresh medium before addition of ligand (10⁻⁸M ^{E2}) or vehicle. After 40 hrs, cells were harvested and extracts analysed for CAT and β-galactosidase activities ^{14,21}, β-galactosidase activities were used to correct for differences in transfection efficiency.

### Example 8

### Pharmaceutical Composition

The following illustrates a representative pharmaceutical dosage form containing a peptide inhibitor and which may be used for therapy.

### Injectable solution

A sterile aqueous solution, for injection, containing per ml of solution:

| | |
|---|---|
| Peptide P-1 | 5.0mg |
| Sodium acetate trihydrate | 6.8mg |
| Sodium chloride | 7.2mg |
| Tween 20^{™} | 0.05mg |

A typical dose of peptide for adult humans is 30mg.

### Example 9

### The strength of the interaction of coactivator signature motifs to NRs varies depending on the precise motif sequence

The interaction between the ERα LBD and a range of different LXXLL motif fusion proteins appeared to yield different reporter gene activity indicating that the ERα LBD interacted with the LXXLL motifs with differing affinities (see Example 5), therefore the strength of these interactions was investigated more closely. The motifs of SRC-1a were tested for their relative interaction specificities with the glucocorticoid receptor and estrogen receptor isoforms α and β in a yeast two hybrid assay.

The SRC-1a motifs 1-4 (SEQ ID 73-76) were expressed as fusion proteins with the LexA DNA binding domain. Motif fusion proteins were generated by ligation of annealed oligonucleotide pairs in frame into the ADH promoter driven LexA DBD vector YCp14-ADH-LexA. YCp14ADH1-LexA is a plasmid from which LexA is expressed under control of the *S. cerevisiae* ADH1 promoter. The backbone of this vector is the plasmid RS314 (Sikorski and Hieter, 1989). Between the Sac I and Kpn I restriction enzyme sites in the polylinker of this vector we have placed an expression cassette comprising the promoter of the *S*. *cerevisiae* ADH1 gene, the coding region of the *E*. *coli* LexA gene and the transcription termination region region of the *S. cerevisiae* ADH1 gene. The promoter region of ADH1 consists of a 1.4kb Bam HI- Hind III fragment from the plasmid pADNS (Colicelli *et al,* 1989). Following the Hind III site is the coding region (amino acids 1-202) of *E. coli* LexA (Horii *et al,* 1981; Miki *et al,* 1981; Markham *et al,* 1981). The *E. coli* LexA fragment was obtained as a Hind III- Pst I fragment from the plasmid pBXL1 (Martin *et al,* 1990). This sequence corresponds to nucleotides 95-710 of Genbank entry g146607. Following this sequence is a region which encodes a polylinker (SEQ ID 77).
GAATTCCTGCAGCCCGGGGTCGACACTAGTTAACTAGCGGCCGC

This polylinker adds the amino acids EFLQPGVDTS (SEQ ID NO: 80) to the carboxy terminus of LexA. The Not I site at the end of this linker is linked to a DNA fragment which includes the transcription terminator region of the *S. cerevisiae* ADH 1 gene. This fragment is the 0.6kb Not I-Bam HI from the plasmid pADNS (Colicelli *et al,* 1989).

The NR LBDs were expressed as fusions with the Gal4 transcriptional AD. The LBD fusions were constructed by cloning a PCR fragment encoding the amino acids corresponding to the LBDs into YCp15Gal1-r11.Ycp15Gal1-r11 is a plasmid from which fusions of the activation region of the *S. cerevisiae* Gal4 protein may be expressed under the control of the *S. cerevisiae GAL1* promoter. The backbone of this vector is the plasmid RS315 (Sikorski and Hieter, 1989). Between the Sac I and Kpn I restriction enzyme sites in the polylinker of this vector we have placed an expression cassette comprising the promoter of the *S. cerevisiae GAL1* gene, the coding region of the fusion protein and the transcriptional termination region of the *S. cerevisiae* ADH1 gene. The *GAL1* promoter (Johnson and Davis, 1984;Yocum *et al,* 1984; West *et al,* 1984) was obtained by amplification of a *S. cerevisiae* genomic fragment by the polymerase chain reaction (PCR). This fragment corresponds to nucleotides 177 to 809 of Genbank database entry g171546. This is followed by the sequence
AAGCTTCCACCATGGTGCCAAAGAAGAAACGTAAAGTT (SEQ ID 78).

This sequence provides a translation initiation codon and a sequence which encodes the amino acids MVPKKKRKV (SEQ ID NO: 81). The last seven residues of this peptide correspond to a region identified as a nuclear localisation signal in the SV40 T antigen (amino acids 126-132 of Genbank entry g310678: Fiers *et al.* 1978; Reddy *et al,* 1978). This region is linked to sequence encoding the region II transcription activation domain (amino acids 768-881) of Gal4, as defined by Ma and Ptashne (1987). The sequence was isolated by PCR from plasmid pBXGalII (P. Broad unpublished) which is a mammalian version of the yeast expression vector pMA236 (Ma and Ptashne, 1987) and corresponds to nucleotides 2744 to 3085 of Genbank entry g171557 (Laughon and Gesteland (1984). This sequence is followed by the polylinker
TCTAGACTGCAGACTAGTAGATCTCCCGGGGCGGCCGC (SEQ ID 79).

All fusion constructs were fully sequenced. The vectors YCp14-ADH-lexA and YCp15Ga11-r1 replicate as a single copy plasmids in yeast(ARS-CEN) and have TRP1 and LEU2 markers respectively.

The S. *cerevisiae* strain MEY132 (M. Egerton, Zeneca Pharmaceuticals, unpublished, genotype (*Mata leu2-3,112 ura3-52 trpl his4 rmel*) was employed as a host strain. A reporter gene consisting of the E. coli lacZ (β-galactosidase) gene under the control of a promoter containing two binding sites for the lexA protein was integrated at the *ura3* locus of this strain. The reporter gene plasmid, JP159-lexRE was constructed using plasmid JP159 as a backbone (J. Pearlberg, Ph.D. Thesis (1994) Harvard University). This is a shuttle vector which contains the *S*. *cerevisiae URA3* as a marker. The plasmid contains the the E.coli β-galactosidase gene under the control of a minimal promoter containing the TATA box and transcription initiation site from the *S*. *cerevisiae GAL1* promoter (Dixon *et al* 1997). Upstream of this promoter is a terminator from the *GAL11* gene. Between the Xba I and Sal I sites in the promoter of this plasmid a 35 nucleotide sequence corresponding to a naturally occurring binding sites for the LexA protein in the promoter of the colicin E1 gene (Ebina *et al,* 1983) the sequence corresponds to residues 20-54 of Genbank entry g 144345). This sequence contains two LexA operators and is therefore referred to as "2lex". This reporter plasmid was linearised within the *URA3* gene and integrated into the *ura3* locus of MEY132 to give the yeast strain MEY132-lexRE

Transformants containing the 2 hybrid fusion constructs were grown to late log phase in 20ml selective medium (2% glucose and appropriate supplements). They were then diluted into 2% galactose containing medium, in the presence or absence of ligand. As controls each LBD fusion was coexpressed with the LexA DBD lacking the coactivator motifs and similarly each motif LexA DBD fusion was coexpressed with Ga14 AD lacking the NR LBD. The relative expression levels of DBD fusion proteins were determined by immunodetection using a monoclonal antibody which recognises LexA.

The estrogen receptor isoforms α and β relative interaction specificities for the 4 motifs of SRC-1a are ranked as follows, 2>4>1>3 (see Figure 5). Glucocorticoid receptor specificities are as follows 4>1=2=3.

The yeast 2-hybrid system used in this Example utilises a single copy integrated reporter gene construct. This enables a quantitative comparison between the different yeast strains bearing the different SRC1a motifs. The data presented in Figure 5 suggest that motif 3 (SRC 1a, 748-753) interacts very weakly, using this system, with ER. Longer exposures to oestradiol (not shown) do however reveal a significant interaction. It is noted that Figure 1A herein clearly shows an interaction between motif 3 and ER but this interaction is significantly weaker than that seen with the other motifs. Any differences in this regard between Figures 5 and 1A can be explained by experimental design features. For example, vectors used to generate the Figure 5 data were low copy number (centromere containing) vectors, whereas vectors used to generate the Figure 1A data were multicopy (2µ) vectors.

### REFERENCES

1. Beato, M., Herrlich, P. & Schutz, G. *Cell* **83**, 851-857 (1995).
2. Mangelsdorf, D.J., *et al.* Cell 83, 835-839 (1995)
3. Onate, S.A., Tsai, S.Y.. Tsai, M.-J. & O'Malley, B.W. Science 270, 1354-1357 (1995).
4. Kamei, Y., *et al.* Cell 85. 403-414 (1996).
5. Yao, T.-P., Ku, G., Zhou, N., Scully, R. & Livingston, D.M. Proc Natl Acad Sci USA 93, (1996).
6. Voegel, J.J., Heine, M.J.S., Zechel, C., Chambon, P. & Gronemeyer, H. EMBO J 15, (1996).
7. Hong, H., Kohli, K., Trivedi, A., Johnson, D.L. & Stallcup, M.R. Proc Natl Acad Sci USA 93, 4948-4952 (1996).
8. Chakravarti, D., *et al.* Nature 383, 99-103 (1996).
9. Hanstein. *B., et al.* Proc Natl Acad Sci USA 93, 11540-11545 (1996).
10.Cavailles, V., *et al.* EMBO J 14, 3741-3751 (1995).
11.Le Douarin, B., *et al.* EMBO J 14, 2020-2033 (1995).
12.Lee, J.W., Ryan, F., Swaffield, J.C., Johnston, S.A. & Moore, D.D. Nature 374, 91-94, (1995).
13.vom Baur, E., *et al.* EMBO J 15, 119-124 (1996).
14.Danielian, P.S., White, R., Lees, J.A. & Parker, M.G. EMBO J 11,1025-1033 (1992).
15.Bourguet, W., Ruff, M., Chambon, P., Gronemever, H. & Moras, D. Nature 375, 377-3 82 (1995).
16.Renaud, J.-P., *et al.* Nature 378, 681-689 (1995).
17. Wagner, *R.L., et al.* Nature 378, 690-697 (1995).
18.Barettino, D., Ruiz, M.D.M.V. & Stunnenberg, H.G. EMBO J 13, 3039-3049 (1994).
19.Durand, B., et al. EMBO J 13, 5370-5382 (1994).
20.Saatcioglu, F., Bartunek, P., Deng, T., Zenke, M. & Karin, M. Mol. Cell. Biol. 13, 3675-3685 (1993).
21.L'Horset, F., Dauvois, S., Heery, D.M., Cavailles, V. & Parker, M.G. Mol. Cell. Biol. 16, 6029-6036 (1996).
22.Rost, B. & Sander, C. Proc Natl Acad Sci USA 90, 7558-7562 (1993).
23.Kwok, R.P.S., *et al.* Nature 370, 223-226 (1994).
24.Arias, J., *et al.* Nature 370, 226-229 (1994).
25.Le Douarin, B., *et al.* EMBO J 15, 6701-6715 (1996).
26.Yeh, S. & Chang, C. Proc Natl Acad Sci USA 93, 5517-5521 (1996).
27. Yoshinaga. S.K., Peterson, C.L., Herskowitz, I. & Yamamoto, K.R. Science 258, 1598-1604 (1992).
28.Stein, B. & Yang, M.X. Mol & Cell Biology 15, 4971-4979 (1995).
29.Metzger, D., Losson, R., Bornert, J.-M., Lemoine, Y. & Chambon, P. Nucl Acid Res 20, 2813-2817 (1992).
30.Le Douarin, B., Pierrat, B., vom Baur, E., Chambon, P. & Losson, R. Nucl Acid Res 23, 876-878(1995).
31.Cavailles, V., Dauvois. S., Danielian, P.S. & Parker, M.G. Proc Natl Acad Sci USA 91, 10009-10013 (1994).
32.Montano, M.M., Ekena, K., Krueger, K.D., Keller, A.L. & Katzenellenbogen, B.S. Mol. Endocrinol 10, 230 -242 (1996).
33.Eckner, R., *et al.* Genes & Dev 8, 869-884 (1994).
34.Colicelli, J. Birchmeier, C., Michaeli, T., O'Neill, K., Riggs, M. and Wigler, M. (1989) Proc. Natl. Acad. Sci. USA 86, 3599-3603.
35.Dixon, G., Scanlon, D., Cooper, S. and Broad, P. (1997). J. Ster. Biochem. Mol. Biol. 62, 165-171.
36.Ebina, Y., Takahara, Y., Kishi, F., Nakazawa, A., and Brent, R. (1983). J. Biol. Chem. 258, 13258-13621.
37.Fiers W., Contreras R., Haegemann G., Rogiers R., De Voorde A.,van Heuverswyn H., Van Herreweghe J., Volckaert G., Ysebaert M. (1978). Nature 273:113-120
38.Horii T., Ogawa T., Ogawa H.(1981) Cell 23:689-697.
39.Johnston M. and Davis R.W.(1984) Mol. Cell. Biol. 4:1440-1448.
40.Laughon, A. and Gesteland, R.F.(1984). Mol. Cell Biol. 4, 260-267.
41.Ma, J. and Ptashne, M. (1987) Cell 48, 847-853.
42.Markham B.E., Little J.W., Mount D.W. (1981). Nucleic Acids Res. 9:4149-4161
43.Martin, K.J., Lillie, J.W. and Green, M.R. (1990) Nature 346, 147-152.
44.Miki T., Ebina Y., Kishi F., Nakazawa A.(1981) Nucleic Acids Res. 9:529-543.
45.Sikorski, R.S. and Hieter, P. (1989) Genetics 122, 19-27.
46.Yocum, R.R.. Hanley, S., West Jr., R. and Ptashne, M. (1984) Mol. Cell Biol. 4, 1985-1998.

### Peptide Library related references

Abelson, J.N. (ed) (1996) Methods in Enzymology **267:** combinatorial chemistry (Academic Press)
Birnbaum, S. and Mosbach, K. (1992) Curr. Opin. Biotechnol. **3,** 49-54.
Brenner, S. and Lerner, R.A. (1992) Proc. Natl Acad. Sci. USA **89,** 5381-5383.
Colas, P., Cohen, B., Jessen, T., Grishina, 1., McCoy, J. and Brent, R. (1996) Nature **380,** 548-550.
Cunningham, B.C., Lowe, D.G., Li, B., Bennett, B.D. and Wells, J.A. (1994) EMBO J. 13, 2508-2515.
Cwirla, S.E., Balasubramanian, P., Duffin, D.J., Wagstrom, C.R., Gates, C.M., Singer, S.C., Davis, A.M., Tansik, R.L., Mattheakis, L.C., Boytos, C.M., Schatz, P.J., Baccanari. D.P., Wrighton, N.C., Barrett, R.W. and Dower, W.J. (1997) Science **276,** 1696-1699.
Cwirla, S.E., Peters, E.A., Barrett, R.W. and Dower, W.J. (1990) Proc. natl. Acad. Sci. USA **87,** 6378-6832.
Devlin, J.J., Panganiban, L.C. and Devlin, P.E. (1990) Science **249,** 404-406**.**
Fields, S. and Song. O.-K. (1989) Nature **340,** 245-246.
Houghten, R.A. (1993) Trends in Genetics **9,** 235-239
Lam, K.S., Salmon, S.E., Hersh, E.M., Hruby, V.J., Kazmierski, W.M. and Knapp, R.J. (1991) Nature **354, 82-84.**
Markland, W., Ley, A.C., Lee, S.W. and Ladner, R.C. (1996)
Matthews, D.J. and Wells, J.A. (1993) Science **260,** 1113-1117.
Roberts, B.L., Markland, W., Ley, A.C., Kent, R.B., White, D.W., Guterman, S.K. and
Ladner, R.C. (1992) Proc. Natl. Acad. Sci. USA **89, 2429-2433.**
Scott, J.K. (1992) Trends Biochem. Sci. **17,** 241-245.
Scott, J.K. and Smith, G.P. (1990) Science **249,** 386-390.
Smith, M.M., Shi, L. and Navre, M. (1995) J. Biol. Chem. **270,** 6440-6449.
Yang, M., Wu, Z. and Fields. S. (1995) Nuc. Acids Res. **23,** 1152-1156.

## Claims

1. A method for identifying inhibitor compounds capable of reducing the interaction between:
a) a first region which is a signature motif on a nuclear protein, and
b) a second region which is that part of a nuclear receptor which is capable of interacting with the nuclear protein through binding to the signature motif,
wherein:
the nuclear protein is a bridging factor that is responsible for the interaction between a liganded nuclear receptor and a transcription initiation complex involved in regulation of gene expression;
the nuclear receptor is a transcription factor;
the signature motif is B¹XXLL in which B¹ is any natural hydrophobic amino acid, L is leucine and X independently represents any natural amino acid which is the key structural element of a nuclear protein which binds to a liganded nuclear receptor as part of the process of the activation or repression of target genes; and
in which the method comprises taking:
i) the potential inhibitor compound;
ii) the liganded nuclear receptor or a fragment thereof in which the fragment comprises the second region defined in this claim in b) above;
iii) a fragment comprising a signature motif of the nuclear protein with the proviso that a fragment which at least includes residues 624-1131 of TIF-2 is excluded; and
iv) detecting the presence or absence of inhibition of the interaction between ii) and iii).

2. A method according to claim 1 in which the fragment of the nuclear protein comprises only one signature motif.

3. A method according to claim 1 or 2 in which B¹ is leucine or valine.

4. A method according to claim 3 in which B¹ is leucine.

5. A method according to any one of claims 1-4 in which the signature motif is further defined as B²B¹XXLL wherein B² is a hydrophobic amino acid.

6. A method according to claim 5 in which B² is selected from the group consisting of isoleucine, leucine, methionine, phenylalanine, tryptophan, tyrosine and valine.

7. A method according to any one of claims 1-6 in which the nuclear protein is a coactivator.

8. A method according to claim 7 in which the coactivator is selected from the group consisting of RIP 140, SRC-1, TIF2, CBP, p300, TIF1, Trip1, Trip2, Trip3, Trip4, Trip5, Trip8, Trip9, p/CIP, ARA70 & Trip230.

9. A method according to any one of claims 1-6 in which the transcription factor is a steroid hormone receptor.

10. A method according to claim 9 in which the steroid hormone receptor is selected from the group consisting of oestrogen receptor, progesterone receptor, androgen receptor and glucocorticoid receptor.

11. A method according to claim 10 in which the steroid hormone receptor is oestrogen receptor.

12. A method according to any preceding claim wherein the method is in the form of a 2-hybrid assay system

13. A method according to any preceding claim wherein the potential inhibitor is a member of a peptide library based on a signature motif as defined in any one of claims 2-6.

14. A peptide selected from the group consisting of PQAQQKSLLQQLLT (SEQ ID NO: 2), KLVQLLTTT (SEQ ID NO: 3), ILHRLLQE (SEQ ID NO: 4) and LLQQLLTE (SEQ ID NO: 5).

15. A pharmaceutical composition which comprises a peptide as defined in claim 14 or a pharmaceutically-acceptable salt thereof, in association with a pharmaceutically-acceptable diluent or carrier.

## Patentansprüche

1. Verfahren zur Identifizierung von Inhibitorverbindungen, die zur Verringerung der Wechselwirkung zwischen:
a) einem ersten Bereich, bei dem es sich um ein Kennmotiv auf einem nukleären Protein handelt, und
b) einem zweiten Bereich, bei dem es sich um denjenigen Teil eines nukleären Rezeptors handelt, der zur Wechselwirkung mit dem nukleären Protein über die Bindung an das Kennmotiv fähig ist,
in der Lage ist, wobei es sich bei
dem nukleären Protein um einen Brückenfaktor handelt, der für die Wechselwirkung zwischen einem mit einem Liganden versehenen nukleären Rezeptor und einem Transkriptionsinitiations-Komplex, der an der Steuerung der Genexpression beteiligt ist, verantwortlich ist;
dem nukleären Rezeptor um einen Transkriptionsfaktor handelt;
dem Kennmotiv um B¹XXLL handelt, worin B¹ für eine beliebige natürliche hydrophobe Aminosäure, L für Leucin und X jeweils unabhängig für eine beliebige natürliche Aminosäure, die das entscheidende Strukturelement eines als Teil des Vorgangs der Aktivierung oder Repression von Zielgenen an einen mit einem Liganden versehenen nukleären Rezeptor bindenden nukleären Proteins darstellt, steht; und
wobei man in dem Verfahren:
i) die potentielle Inhibitorverbindung,
ii) den mit einem Liganden versehenen nukleären Rezeptor oder ein Fragment davon, wobei das Fragment den zweiten Bereich mit der in diesem Anspruch oben unter b) angegebenen Bedeutung umfaßt,
iii) ein ein Kennmotiv des nukleären Proteins umfassendes Fragment mit der Maßgabe, daß dabei ein Fragment, das mindestens die Reste 624-1131 von TIF-2 enthält, ausgeschlossen ist, nimmt und
iv) das Vorhandensein oder die Abwesenheit einer Hemmung der Wechselwirkung zwischen ii) und iii) nachweist.

2. Verfahren nach Anspruch 1, wobei das Fragment des nukleären Proteins nur ein Kennmotiv umfaßt.

3. Verfahren nach Anspruch 1 oder 2, wobei es sich bei B¹ um Leucin oder Valin handelt.

4. Verfahren nach Anspruch 3, wobei es sich bei B¹ um Leucin handelt.

5. Verfahren nach einem der Ansprüche 1-4, wobei das Kennmotiv ferner als B²B¹XXLL definiert ist, wobei es sich bei B² um eine hydrophobe Aminosäure handelt.

6. Verfahren nach Anspruch 5, wobei B² ausgewählt ist aus der Gruppe bestehend aus Isoleucin, Leucin, Methionin, Phenylalanin, Tryptophan, Tyrosin und Valin.

7. Verfahren nach einem der Ansprüche 1-6, wobei es sich bei dem nukleären Protein um einen Coaktivator handelt.

8. Verfahren nach Anspruch 7, wobei der Coaktivator ausgewählt ist aus der Gruppe bestehend aus RIP 140, SRC-1, TIF2, CBP, p300, TIF1, Trip1, Trip2, Trip3, Trip4, Trip5, Trip8, Trip9, p/CIP, ARA70 & Trip230.

9. Verfahren nach einem der Ansprüche 1-6, wobei es sich bei dem Transkriptionsfaktor um einen Steroidhormonrezeptor handelt.

10. Verfahren nach Anspruch 9, wobei der Steroidhormonrezeptor ausgewählt ist aus der Gruppe bestehend aus Östrogenrezeptor, Progesteronrezeptor, Androgenrezeptor und Glucocorticoidrezeptor.

11. Verfahren nach Anspruch 10, wobei es sich bei dem Steroidhormonrezeptor um Östrogenrezeptor handelt.

12. Verfahren nach einem vorhergehenden Anspruch, wobei das Verfahren die Form eines 2-Hybrid-Assay-Systems hat.

13. Verfahren nach einem vorhergehenden Anspruch, wobei es sich bei dem potentiellen Inhibitor um ein Mitglied einer auf einem Kennmotiv mit der in einem der Ansprüche 2-6 angegebenen Bedeutung beruhenden Peptidbibliothek handelt.

14. Peptid, ausgewählt aus der Gruppe bestehend aus PQAQQKSLLQQLLT (SEQ ID NO: 2), KLVQLLTTT (SEQ ID NO: 3), ILHRLLQE (SEQ ID NO: 4) und LLQQLLTE (SEQ ID NO: 5) .

15. Pharmazeutische Zusammensetzung, die ein Peptid mit der in Anspruch 14 angegebenen Bedeutung oder ein pharmazeutisch annehmbares Salz davon in Verbindung mit einem pharmazeutisch annehmbaren Verdünnungs- oder Trägermittel umfaßt.

## Revendications

1. Procédé d'identification de composés inhibiteurs capables de réduire l'interaction entre :
a) une première région qui est un motif de signature sur une protéine nucléaire, et
b) une deuxième région qui est la partie d'un récepteur nucléaire qui est capable d'interagir avec la protéine nucléaire par liaison au motif de signature, dans lequel
la protéine nucléaire est un facteur de pontage qui est responsable de l'interaction entre un récepteur nucléaire à ligand et un complexe d'initiation de la transcription impliqué dans la régulation de l'expression génique,
le récepteur nucléaire est un facteur de transcription,
le motif de signature est B¹XXLL, dans lequel B¹ est n'importe quel acide aminé hydrophobe naturel, L est leucine et X représente indépendamment n'importe quel acide aminé naturel qui est l'élément structurel clé d'une protéine nucléaire qui se lie à un récepteur nucléaire à ligand dans le cadre du procédé de l'activation ou de la répression de gènes cibles, et
dans lequel le procédé comprend la prise :
i) du composé inhibiteur potentiel,
ii) du récepteur nucléaire à ligand ou d'un fragment de celui-ci, où le fragment comprend la deuxième région définie dans cette revendication en b) ci-dessus,
iii) d'un fragment comprenant un motif de signature de la protéine nucléaire à la condition qu'un fragment qui comprend au moins les résidus 624-1131 de TIF2 soit exclu, et
iv) la détection de la présence ou de l'absence de l'inhibition de l'interaction entre ii) et iii).

2. Procédé selon la revendication 1, dans lequel le fragment de la protéine nucléaire comprend seulement un motif de signature.

3. Procédé selon la revendication 1 ou 2, dans lequel B¹ est leucine ou valine.

4. Procédé selon la revendication 3, dans lequel B¹ est leucine.

5. Procédé selon l'une quelconque des revendications 1 à 4, dans lequel le motif de signature est en outre défini comme étant B²B¹XXLL, dans lequel B² est un acide aminé hydrophobe.

6. Procédé selon la revendication 5, dans lequel B² est choisi dans le groupe constitué de l'isoleucine, de la leucine, de la méthionine, de la phénylalanine, du tryptophane, de la tyrosine et de la valine.

7. Procédé selon l'une quelconque des revendications 1 à 6, dans lequel la protéine nucléaire est un coactivateur.

8. Procédé selon la revendication 7, dans lequel le coactivateur est choisi dans le groupe constitué de RIP 140, de SRC-1, de TIF2, de CBP, de p300, de TIF1, de Trip1, de Trip2, de Trip3, de Trip4, de Trip5, de Trip8, de Trip9, de p/CIP, d'ARA70 et de Trip230.

9. Procédé selon l'une quelconque des revendications 1 à 6, dans lequel le facteur de transcription est un récepteur à hormone stéroïdienne.

10. Procédé selon la revendication 9, dans lequel le récepteur à hormone stéroïdienne est choisi dans le groupe constitué d'un récepteur à oestrogène, d'un récepteur à progestérone, d'un récepteur à androgène et d'un récepteur à glycocorticoïdes.

11. Procédé selon la revendication 10, dans lequel le récepteur à hormone stéroïdienne est un récepteur à oestrogène.

12. Procédé selon l'une quelconque des revendications précédentes, dans lequel le procédé est sous la forme d'un système de test à deux hybrides.

13. Procédé selon l'une quelconque des revendications précédentes, dans lequel l'inhibiteur potentiel est un membre d'une banque peptidique basée sur un motif de signature tel que défini dans l'une quelconque des revendications 2 à 6.

14. Peptide choisi dans le groupe constitué de PQAQQKSLLQQLLT (SEQ ID NO 2), KLVQLLTTT (SEQ ID NO 3), ILHRLLQE (SEQ ID NO 4) et LLQQLLTE (SEQ ID NO 5).

15. Composition pharmaceutique qui comprend un peptide tel que défini dans la revendication 14 ou un sel pharmaceutiquement acceptable de celui-ci, en association avec un diluant ou un support pharmaceutiquement acceptable.
